# EUROPÄISCHE PATENTSCHRIFT

(11) **EP 0 724 872 B1**
(45) Veröffentlichungstag und Bekanntmachung des Hinweises auf die Patenterteilung: **23.04.2003**
(21) Anmeldenummer: 95250237.5
(22) Anmeldetag: 28.09.1995
(51) Int. Cl.: A61H 7/00, A46B 13/04

(54) **Motorbetriebenes Massagehandgerät**
Motor driven massage appliance
Dispositif de massage motorisé

(30) Priorität: 04.02.1995 DE 19504761
(43) Veröffentlichungstag der Anmeldung: 07.08.1996
(73) Patentinhaber: Groenewold, Vera, 14193 Berlin (DE)
(72) Erfinder: Groenewold, Vera, D-14193 Berlin (DE); Schmett, Michael, D-14089 Berlin (DE)
(74) Vertreter: Lüke, Dierck-Wilm, Dipl.-Ing.

(56) Entgegenhaltungen:
- CH-A- 616 075
- DE-A- 4 306 242
- DE-C- 825 135
- FR-A- 1 225 094
- FR-A- 1 565 296
- GB-A- 1 480 265
- US-A- 5 385 532

## Beschreibung

Die Erfindung bezieht sich auf motorbetriebenes Massagehandgerät gemäß dem Oberbegriff des Patentanspruches 1. Derartige Geräte werden für kosmetische und medizinische Zwecke eingesetzt, u.a. zur Behandlung rheumatischer Erkrankungen.

Ein motorbetriebenes Massagehandgerät der gattungsgemäßen Art ist aus der US-A-5 385 532 vorbekannt. Bei diesem wird die Massagesubstanz durch einen im Zentrum des Gehäuses befindlichen Kanal mittels eines Druckluftsystems herausgedrückt und über eine im Zentrum des Gehäuses angeordnete Sickerkappe ausgegeben. Die Verteilung der Massagesubstanz erfolgt dann über drei umlaufende, rotierende Bürsten. Nachteilig hierbei sind die gesondert erforderliche manuelle Handhabung der Zufuhr der Massagesubstanz über die Drucklufteinrichtung und die gesondert erforderliche, manuell erfolgende genaue Dosierung der Massagesubstanz.

Bei einem gattungsfremden Massagehandgerät nach DE-A-43 06 242 ist nur eine einzige Massagebürste vorhanden, welcher die Massagesubstanz aus einer außerhalb des Massagehandgerätes befindlichen Flasche manuell zugesetzt wird. Nachteilig hierbei sind auch die gesondert erforderliche manuelle Handhabung der Massagesubstanz sowie die Dosierung der Massagesubstanz.
Aus der DE-A-28 30 480 ist schließlich ein Haut- und/oder Haarkosmetikgerät vorbekannt, das hauptsächlich zum Auf- und Einbringen von Flüssigkeiten in das Kopfhaar vorgesehen ist und einen von Kanälen durchzogenen Bürstenteil aufweist, die über eine Leitung mit einem Vorratsbehälter für gefärbte Flüssigkeiten verbunden sind. Die Flüssigkeit wird durch Vibration im Kopfhaar verteilt. Nachteilig hierbei sind insbesondere die geringe, erzielbare Massagewirkung sowie die schlechte Dosierungsmöglichkeit für die Flüssigkeit.

Der Erfindung liegt die Aufgabe zugrunde, das motorbetriebene Massagehandgerät der gattungsgemäßen Art im Hinblick auf eine wirksamere Massagewirkung und eine einfache Dosierung der Massagesubstanz zu verbessern.

Die Lösung dieser Aufgabe ergibt sich aus den kennzeichnenden Merkmalen des Patentanspruches 1. Es ist einerseits eine Mehrzahl von rotierenden Massagebürsten vorgesehen, wodurch die Massagewirkung verbessert wird. Es ist andererseits eine direkte Zuführung der Massagesubstanz zu den einzelnen Bürsten über im Gehäuse angeordnete Bohrungen, wodurch die Dosierung verbessert wird. De Zufuhr der Massagesubstanz erfolgt in steuerbaren Intervallen, wodurch die Dosierung der Massagesubstanz noch genauer bewirkt wird. Die Mehrzahl der Massagebürsten gewährleistet durch einen Walkeffekt der Haut eine optimale Massagewirkung, die noch durch die präzise Dosierung der Massagesubstanz zusätzlich beeinflußt wird. Die Zufuhr der Massagesubstanz kann beliebig zu- und abgeschaltet werden.

Gemäß der Erfindung erfolgt die Zufuhr der Massagesubstanz nacheinander jeweils über eine der aufeinanderfolgend rotierenden Bürsten und in steuerbaren Intervallen, wodurch die Dosierung der Massagesubstanz noch genauer erfolgen kann.

Mit dem erfindungsgemäßen, motorbetriebenen Massagehandgerät können sowohl kosmetische als auch medizinische Behandlungen mit unterschiedlichen Massagesubstanzen ausgeführt werden.

Die Erfindung ist nachfolgend anhand eines in den Zeichnungen dargestellten Ausführungsbeispieles eines Massagehandgerätes näher erläutert. Es zeigen:
- Fig. 1: einen axialen Längsschnitt,
- Fig. 2: einen Längsschnitt gemäß der Fig. 1 entlang der Linie A-A und
- Fig. 3: einen teilweisen Längsschnitt gemäß der Fig.1 entlang der Linie B-B.

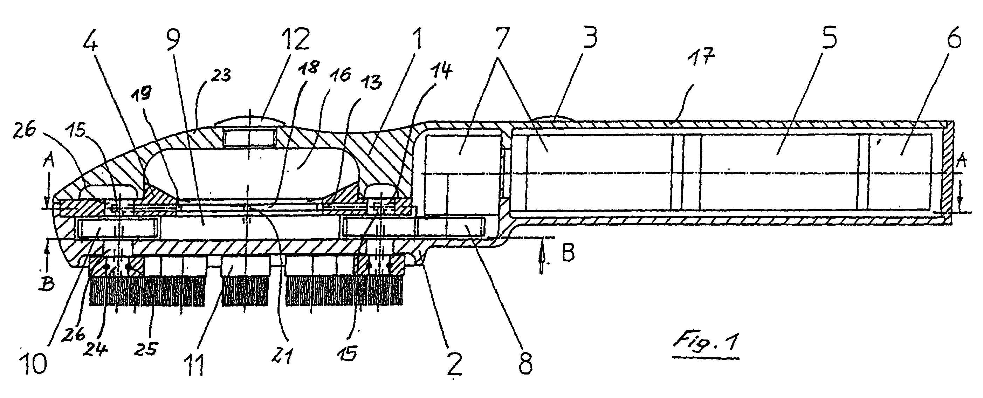

Die Fig. 1 zeigt im axialen Längsschnitt die wesentlichen Baugruppen des Massagehandgerätes. Danach besteht das Massagehandgerät aus einem aus Oberteil 1 und Unterteil 2 gebildeten Gehäuse, in dessen Griffteil 17 ein Motor 5 mit Akku 6, ein Getriebe 7 und ein Antriebszahnrad 8 angeordnet sind. Im Kopfteil 23 sind eine kreisscheibenförmige Ölpumpe 9, eine oberhalb derselben angeordnete Vorratskammer 16 für Massagesubstanzen und radial um die Ölpumpe 9 zwölf Zahnräder 10 eingebracht. Auf der Mantelfläche des Griffteiles 17 sind zwei Taster 3 für die Ein-/Aus-Schaltung des Motors 5 und der Ölpumpe 9 und auf dem Kopfteil 23 eine Verschlußschraube 12 für die Ölvorratskammer 16 vorgesehen. Die Stirnseite des Griffteiles 17 enthält eine nicht dargestellte Steckverbindung, über die ein Netzkabel zur Akkuaufladung oder zum direkten Antrieb des Motors 5 angeschlossen werden kann. Im Kopfteil 23 des Gehäuses ist zwischen dem Ober- und dem Unterteil 1,2 eine Zwischenplatte 4 eingebracht, in der die Zahnräder 10 gelagert sind, an deren Unterseite Massagebürsten 11 angebracht sind.

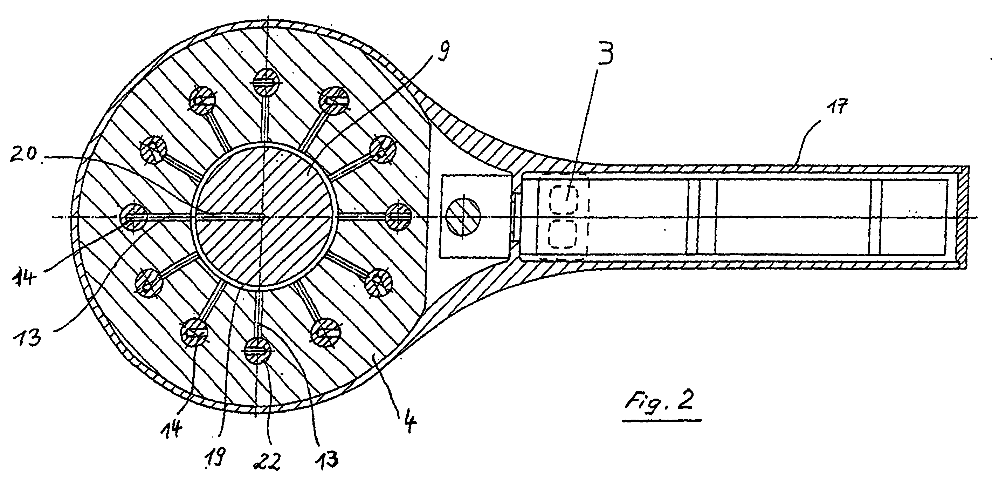

Entsprechend der Schnittdarstellung in Fig. 2 weist die Zwischenplatte 4 sternförmig angeordnete Radialbohrungen 13 auf, die von der Mitte der Zwischenplatte 4 zu den Lagerstellen 22 der Zahnräder 10 führen und zur Ölzuführung an die auf die Zahnräder 10 aufgesetzten Bürsten 11 dienen. Die Zahnräder 10 sind mit ihren Paßsitzen 26 in der Zwischenplatte 4 und im Gehäuseunterteil 2 drehbar gelagert (Fig. 1).

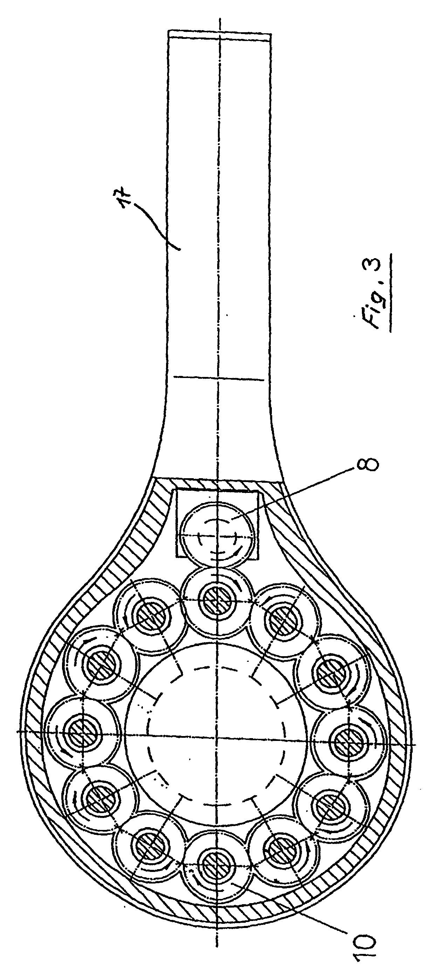

Wie die Fig. 3 zeigt, sind die Zahnräder 10 auf einem gemeinsamen Teilkreis angeordnet und befinden sich mit dem jeweiligen Folgezahnrad 10 im Eingriff. Der Antrieb erfolgt über das Antriebszahnrad 8, welches mit dem vom Motor 5 angetriebenen Getriebe 7 (Fig. 1,2) fest verbunden ist. Die Drehrichtung der miteinander im Eingriff stehenden Zahnräder 10 ist abwechselnd gegensinnig, wie es in Fig. 3 mit den Pfeilen dargestellt ist.

Wie die Fig. 1 zeigt, sind die Zahnräder 10 auf der Gehäuseunterseite mit einem Vierkant 24 und einer Ringnut 25 versehen, die eine axiale und radiale Bürstenaufnahme gewährleisten. Die Zahnräder 10 sind mit radialen Sacklochbohrungen 14 und mit den Sacklochbohrungen 14 verbundenen Bohrungen 15 versehen, über die das Öl an die Bürsten 11 gefördert wird. Die Stellung der Zahnräder 10 zueinander ist so gewählt, daß immer nur eine Bürste 11 mit Öl versorgt werden kann. Dies wird durch die verschiedenen radialen Bohrungsanordnungen 14 in den Zahnrädern 10 erreicht (Fig.2). Im Zentrum der Zwischenplatte 4 ist die Ölpumpe 9 angeordnet, deren Ansaugöffnung 21 in die Ölvorratskammer 16 mündet (Fig. 1). Ein umlaufender Ringkanal 19 am Pumpenflansch 18 versorgt über die Querbohrungen 13 der Zwischenplatte 4 die Bürsten 11 (Fig.1,2).

Im folgenden wird die Funktionsweise des Massagehandgerätes beschrieben.

Der Antrieb der Massagebürsten 11 erfolgt über die aus dem Motor 5, dem Getriebe 7, dem Antriebszahnrad 8 und den Zahnrädern 11 gebildete Antriebseinheit. Die Antriebsleistung des Motors 5 wird über das Getriebe 7, welches mit dem Antriebszahnrad 8 fest verbunden ist, als Rotationsbewegung auf die Zahnräder 10 mit den aufgesetzten Bürsten 11 übertragen. Der Motor 5 wird direkt über ein Netzkabel oder über den Akku 6 betrieben. Über den Bedientaster 3 auf der Mantelfläche des Griffteils 17 wird der Motor 5 einund ausgeschaltet (Fig. 1). Durch die entsprechend der Fig. 3 auf einem gemeinsamen Teilkreis angeordneten Zahnräder 10 werden die Bürsten 11 in gegensinniger Drehrichtung zueinander bewegt, was auf der massierten Hautpartie einen Walkeffekt bewirkt.

Die Miniaturölpumpe 9 saugt das Öl über die Ansaugöffnung 21 aus der Vorratskammer 16 an und verteilt es über einen Ringkanal-Pumpenauslaß 20 und den Ringkanal 19 sowie über die Bohrungen 13 der Zwischenplatte 4 an die Bohrungen 14,15 in den Zahnrädern 10 und zu den Bürsten 11 (Fig.1,2). Die Ölpumpe 9 wird nach Bedarf über den Bedientaster 3 zugeschaltet. In der Vorratskammer 16 sind ca. 50 ml Massageöl. Um eine gleichbleibende Öldosierung an den einzelnen Bürsten 11 zu erreichen, sind die radialen Bohrungen 14 der Zahnräder 10 versetzt zueinander angeordnet. Es ist immer nur eine Zahnradbohrung 14 mit der Bohrung 13 der Zwischenplatte 4 deckungsgleich, alle anderen Sacklochbohrungen 14 sind verschlossen (Fig. 2). Bei jeder Getriebeumdrehung werden alle Zahnräder 10 und Bürsten 11 nacheinander mit Öl versorgt.

Ein leichter Wechsel der Bürsten 11 wird durch die verdrehsichere Vierkantverbindung mit den Zahnrädern 10 gewährleistet. Bürste 11 und Zahnrad 10 sind jeweils axial über einen Rastmechanismus fixiert (Fig.1).

Der Griff 17 des Gehäuses kann durch eine nicht dargestellte aufgesteckte oder aufgeschraubte Griffverlängerung so gestaltet werden, daß mit dem Massagegerät alle Körperpartien erreicht werden.

### BEZUGSZEICHENLISTE

- 1: Oberteil
- 2: Unterteil
- 3: Bedientaster
- 4: Zwischenplatte
- 5: Motor
- 6: Akku
- 7: Getriebe
- 8: Antriebszahnrad
- 9: Ölpumpe
- 10: Zahnrad
- 11: Bürste
- 12: Verschlußschraube
- 13: Bohrung
- 14: Bohrung
- 15: Bohrung
- 16: Vorratskammer
- 17: Griffteil
- 18: Pumpenflansch
- 19: Ringkanal
- 20: Ringkanal-Pumpenauslaß
- 21: Ansaugöffnung
- 22: Lagerstelle
- 23: Kopfteil
- 24: Vierkant
- 25: Ringnut
- 26: Paßsitz

## Patentansprüche

1. Motorbetriebenes Massagehandgerät zur Körpermassage mittels rotierender Massagebürsten (11), aus einem Gehäuse (1,2) mit einem Griffteil (17), einer Vorratskammer (16) für eine kosmetische oder medizinische Massagesubstanz und Mitteln (9,10,13-15,19-21) zur Zuführung der Massagesubstanz, wobei
eine Mehrzahl von rotierenden Massagebürsten (11) vorgesehen ist
**dadurch gekennzeichnet,**
**daß** die Massagesubstanz über die Mittel (9,10,13-15, 19-21) den einzelnen Massagebürsten (11) über im Gehäuse (1,2) angeordnete Bohrungen (13) nacheinander zuführbar ist, wobei die Massagebürsten (11) über Zahnräder (10) mit radialen und axialen Bohrungen (14,15) und über die sternförmig in einer Zwischenplatte (4) angeordneten Bohrungen (13), die jeweils nur mit einer der zueinander versetzt angeordneten Radialbohrungen (14) der Zahnräder (10) deckungsgleich sind, und über eine Pumpe (9) mit der Vorratskammer (16) für die Massagesubstanz verbunden sind.

2. Massagehandgerät nach Anspruch 1, **dadurch gekennzeichnet, daß** die rotierenden Massagebürsten (11) auf einem gemeinsamen Teilkreis und zueinander gegensinnig rotierend angeordnet sind.

3. Massagehandgerät nach Anspruch 1 oder 2, **dadurch gekennzeichnet, daß** die Zahnräder (10) mit ihren radialen Bohrungen (14) bezüglich der Stellung der Bohrungen (14) zueinander unterschiedlich angeordnet sind und jeweils nur eine Massageürste (11) mit der Massagesubstanz versorgt ist.

4. Massagehandgerät nach Anspruch 3, **dadurch gekennzeichnet,daß** die Pumpe (9) im Zentrum der Zwischenplatte (4) angeordnet ist und die Ansaugöffnung (21) der Pumpe (9) in die Vorratskammer (16) mündet wobei am Pumpenflansch (18) ein mit den Bohrungen (13) der Zwischenplatte (4) verbundener, umlaufender Ringkanal (19) vorgesehen ist.

5. Massagehandgerät nach Anspruch 4, dadurch gekenn zeichnet, daß die Zwischenplatte (4) zwischen Oberund Unterteil (1,2) des Gehäuses angeordnet ist und die Zahnräder (10) in der Zwischenplatte (4) und im Unterteil (2) drehbar gelagert und über ein Antriebszahnrad (8) vom Motor (5) angetrieben sind und mit dem jeweiligen Folgezahnrad (10) auf einem gemeinsamen Teilkreis in Eingriff stehen.

## Claims

1. A motor driven massage appliance for body massaging by means of rotating massage brushes (11), comprising a housing (1, 2) with a handle portion (17), a storage chamber (16) for a cosmetic or medical massage substance and means (9, 10, 13-15, 19-21) for supplying said massage substance, a multitude of rotating massage brushes (11) being provided, **characterized by** that the massage substance can successively be supplied by the means (9, 10, 13-15, 19-21) to the individual massage brushes (11) through boreholes (13) arranged in the housing (1, 2), the massage brushes (11) being connected by gears (10) with radial and axial boreholes (14, 15) and by the boreholes (13) disposed in a star-type manner in an intermediate panel (4), said boreholes coinciding with one only of the staggered radial boreholes (14) of the gears (10), and being connected by a pump (9) with the storage chamber (16) for the massage substance.

2. A motor driven massage appliance according to claim 1, **characterized by** that the rotating massage brushes (11) are arranged on a common pitch circle, and are rotating in an opposed direction to each other.

3. A motor driven massage appliance according to claim 1 or 2, **characterized by** that the gears (10) with their radial boreholes (14) are arranged in a different manner from each other, with regard to the boreholes (14), and that one massage brush (11) only is respectively supplied with the massage substance.

4. A motor driven massage appliance according to claim 3, **characterized by** that the pump (9) is located in the center of the intermediate panel (4), and the suction aperture (21) of the pump (9) terminates in the storage chamber (16), at the pump flange (18) being provided a circumferential annular channel (19) connected to the boreholes (13) of the intermediate panel (4).

5. A motor driven massage appliance according to claim 4, **characterized by** that the intermediate panel (4) is disposed between the upper and lower portions (1, 2) of the housing, and the gears (10) are rotatably supported in the intermediate panel (4) and in the lower portion (2) and are driven via a drive gear (8) by the motor (5) and are in engagement with the respective following gear (10) on a common pitch circle.

## Revendications

1. Dispositif de massage motorisé pour le massage du corps au moyen de brosses de massage rotatives (11), comprenant un boîtier (1, 2) avec une poignée (17), une chambre de stockage (16) pour une substance de massage cosmétique ou médicinale et des moyens (9, 10, 13-15, 19-21) pour l'alimentation de la substance de massage, une multiplicité de brosses de massage rotatives (11) étant prévue, **caractérisé en ce que** la substance de massage est alimentable successivement par l'intermédiaire des moyens (9, 10, 13-15, 19-21) à travers de forures (13) agencées dans le boîtier (1, 2), les brosses de massage (11) étant liées par l'intermédiaire de roues dentées (10) avec des forures radiales et axiales (14, 15) et par l'intermédiaire des forures (13) agencées en forme d'étoile dans une plaque intermédiaire (4) et coïncidentes avec respectivement seulement une des forures radiales (14) décalées des roues dentées (10) et par l'intermédiaire d'une pompe (9) avec la chambre de stockage (16) pour la substance de massage.

2. Dispositif de massage selon la revendication 1, **caractérisé en ce que** les brosses de massage rotatives (11) sont agencées sur un cercle primitif de référence et en sens inverse.

3. Dispositif de massage selon la revendication 1 ou 2, **caractérisé en ce que** les roues dentées (10) sont agencées avec leurs forures radiales (14) en manière différente l'une de l'autre par rapport à la position des forures (14), et seulement une brosse de massage (11) est respectivement alimentée de substance de massage.

4. Dispositif de massage selon la revendication 3, **caractérisé en ce que** la pompe (9) est agencée au centre de la plaque intermédiaire (4), et que l'orifice d'aspiration (21) de la pompe (9) débouche dans la chambre de stockage (16), à la bride (18) de la pompe étant prévu un canal ceinture (19) périphérique lié aux forures (13) de la plaque intermédiaire (4).

5. Dispositif de massage selon la revendication 4, **caractérisé en ce que** la plaque intermédiaire (4) est agencée entre la partie supérieure et inférieure (1, 2) du boîtier, et que les roues dentées (10) sont pivotantes dans la plaque intermédiaire (4) et dans la partie inférieure (2) et entraînées par l'intermédiaire d'une roue dentée motrice (8) par le motuer (5) et s'engrènent avec la roue dentée (10) respectivement suivante sur un cercle primitif de référence commun.
